Europäisches Patentamt

European Patent Office

Office européen des brevets

(11) Publication number: **0 517 199 A1**

## EUROPEAN PATENT APPLICATION

(21) Application number: **92109402.5**

(22) Date of filing: **03.06.92**

(51) Int. Cl.⁵: **C12N 15/00**, G01N 33/574, A01K 67/027

(30) Priority: **04.06.91 IL 98369**

(43) Date of publication of application:
**09.12.92 Bulletin 92/50**

(84) Designated Contracting States:
**AT BE CH DE DK ES FR GB GR IT LI LU MC NL PT SE**

(71) Applicant: **YEDA RESEARCH AND DEVELOPMENT COMPANY, LTD.**
**Kiryat Weizman P.O. Box 95**
**Rehovot 76100(IL)**

(72) Inventor: **Reisner, Yair**
**4 Mazal Dagim Street**
**Old Yaffo, Tel Aviv(IL)**

(74) Representative: **Vossius & Partner**
**Siebertstrasse 4 P.O. Box 86 07 67**
**W-8000 München 86(DE)**

(54) **Durable engraftment of human tissue and cells in normal mammals.**

(57) A non-human chimeric mammal M4 having long-term stable xenogeneic tissues or cells other than hematopoietic cells, is obtained by doubly transplanting a mammal M1, the hematopoietic cells of which have been substantially suppressed or destroyed, with tissue or cells other than hematopoietic cells originating from a mammal M3 of a species other than that of mammal M1, preferably human, and hematopoietic cells originating from a mammal M2, of a species other than that of M3, preferably of the same species as M1, having a genetically-determined hematopoietic deficiency. In particular, chimeric mice having long term human tissue or malignant cells were obtained by double engraftment of tissue derived from human organs or melanoma cells and of bone marrow cells derived from a mouse with severe combined immunodeficiency (SCID).

EP 0 517 199 A1

The present invention relates to non-human chimeric mammals having long-term stable xenogeneic, preferably human, tissues or cells, and to methods for their production. In particular, it relates to chimeric mice having long-term stable human malignant cells, obtained by double transplant with bone marrow cells from a severe combined immunodeficient (SCID) mouse and with malignant cells from a human donor, which can be used for chemotherapy sensitivity testing of different drugs or combinations thereof.

Immunodeficient animals are being used as experimental models to study hematopoietic disorders. Xenogeneic bone marrow has been successfully transplanted only between closely related species, such as rat and mouse (for review, see: van Bekkum, D.W. and Lowenberg, B. (1985) Bone Marrow Transplantation: Biological Mechanisms and Clinical Practice, Dekker, New York, pp. 311-350). Recently, attempts have been made to produce a mouse with an immune system of human origin. Such animals can be used as models for the study of human hematopoietic cells and the human immune system. An example is the SCID-hu mouse, obtained by transplanting cells from the human immune system into a natural mutant mouse having an inherited condition called "severe combined immunodeficiency" (SCID). (McCune, J.M., et al. (1988) Science **241**, 1632, and Namikawa R., Weilbaecher, K.N., Kaneshima, H., Yee, E.J. and McCune, J.M. (1990) J. Exp. Med. **172**, 1055). Although engraftment of human cells in these models permitted for the first time the infection of mice with HIV (Namikawa, et al., cited above), the survival of human cells was severely limited. Human lymphocytes were not detected in significant numbers in the mouse's peripheral blood beyond 2-3 months post-transplant. Moreover, in McCune's model, hematopoietic stem cells from human fetal liver transferred into SCID mice did not engraft or differentiate into T cells unless supplemented with a graft of human fetal thymic epithelium implanted under the kidney capsule. Another mouse model is based on the transplantation of human hematopoietic stem cells into the genetically immunodeficient Bg/Nu/Xid mouse. Here too, human lymphocytes did not induce graft-versus-host disease (Kamel-Reid, S. and Dick, J.E. (1988) Science **242**, 1706).

Previous clinical studies have suggested that, following transplantation of T cell-depleted bone marrow (BM) into human SCID (Reisner, Y. et al. (1983) Blood **61**, 341) or leukemic patients (Keever, C.A. et al. (1989) Blood **73**, 1340), differentiation of human stem cells into mature T cells is slow, taking from 1-4 months. T cell differentiation in mice takes about 14-21 days following lethal total body irradiation (TBI) and BM transplantation. Given these differences in the pace of reconstitution, it is clear that in a murine microenvironment, human stem cell differentiation may be inefficient and even slower, due to the poor cross-reactivity between murine and human cytokines and other molecules involved in cellular recognition.

Following heavy suppression of the immune and hematopoietic system, as with lethal TBI, normal mice die within 2 weeks if bone marrow is not provided. Even if human stem cells could theoretically grow in such mice and reconstitute the mouse's hematopoietic system, their slow rate of differentiation into mature immunocompetent lymphocytes or other leucocytes might prevent them from reaching large enough numbers to protect the mice from infections; furthermore, inadequate replenishing of hematopoietic compartments would not protect the mice from death by hematopoietic failure. On the other hand, sublethal conditioning protocols (e.g. sublethal TBI), which spare substantial numbers of hematopoietic and lymphoid cells, would enable endogenous murine cells to compete effectively with transplanted human cells, and ultimately reject the human graft.

The production of stable human-mouse chimeras by transplantation of human bone marrow into lethally irradiated normal mice has been disclosed in European Patent Application published under No. EP 438053 and in Lubin, I. et al., (1991) Science **252**, 427. For production of a useful mouse-human chimeric model, the conflict between the need for prompt hematopoietic reconstitution on the one hand, and the slow differentiation rate of human T and B cells on the other hand, was resolved by using SCID mice as bone marrow donors rather than as bone marrow recipients. Bone marrow cells from SCID mice can promptly reconstitute all hematopoietic lineages except T and B lymphocytic lineages, which cannot develop from SCID pluripotent stem cells due to the deficiency in rearrangement of antigen receptor genes in SCID mice (Schuler, W. et al. (1986) Cell **46**, 963). To test whether the resultant "empty space" in the thymus and bone marrow could gradually be occupied by, and populated with, normal human T and B cells, a transplant of SCID mouse bone marrow was combined with a transplant of human bone marrow that had been depleted of both T and B cells by differential agglutination with soybean agglutinin (SBA⁻) and subsequent removal of residual $CD2^+$ lymphocytes that form rosettes with sheep erythrocytes (E + ⁻) resulting in human BM cells, termed SBA⁻E⁻ (Reisner, Y. et al. (1981) Lancet **ii**, 327).

Thus, a stable long-term human-mouse chimerism was achieved by lethally irradiating normal BALB/c mice, transplanting them with human bone marrow cells depleted of T and B cells, followed one day later by T cell-depleted bone marrow cells from SCID mice. As mentioned above, SCID mice were used as bone marrow donors so that all hematopoietic lineages except the T and B cell lineages would be rapidly reconstituted. Phenotypically mature human T and B cells were initially detected in peripheral blood of transplanted mice 2-4 months post-transplant, and continued to be detected more than 9 months post-transplant. Similar lymphoid chimerism in peripheral blood, without apparent graft vs. host disease, was also found when unmanipulated human bone marrow (i.e., not depleted of T and B lymphocytes) was transplanted either intravenously or intraperitoneally.

Nakamura, T. et al. (1986) Proc. Natl. Acad. Sci. U.S.A. **83**, 4529-4532 described successful liver allografts established by combination with allogeneic bone marrow transplantation. Thus liver pieces from BALB/c mouse were accepted when transplanted under the kidney capsules of lethally irradiated C3H/HeN mice which were reconstituted with bone marrow of BALB/c mice. Most importantly, such chimeric mouse rejected liver from C57BL/6J mouse (differing in their H-2 from the bone marrow donor). Therefore, in this system, tolerance towards liver grafting is confined to the strain of the bone marrow donor. It is clearly desirable to engraft tissue or cells from donors other than the bone marrow donors, including human xenografts, in a non-human mammal, and this is achieved by the present invention.

The present invention is directed to a non-human chimeric mammal M4 having long-term stable xenogeneic tissue or cells, other than normal hematopoietic cells, originating from a mammal M3, said mammal M4 being originated from a mammal M1 whose hematopoietic cells have been substantially suppressed or destroyed and replaced by hematopoietic cells originating from a mammal M2, of a species other than that of M3, having a genetically-determined hematopoietic deficiency, and to which mammal M1 tissue or cells other than normal hematopoietic cells of mammal M3 have been transplanted.

The tissue from mammal M3 is preferably pieces of an organ and the cells are preferably malignant cells. The invention also relates to a chimeric non-human mammal M4 having malignant cells originating from human solid tumors or leukemias and in which the endogenous hematopoietic cells have been replaced by exogenous hematopoietic cells originating from a non-human mammal M2, having a genetically determined hematopoietic deficiency.

In another embodiment, the invention relates to a chimeric non-human mammal M4 having tissue derived from human organs and in which the endogenous hematopoietic cells have been replaced by exogenous hematopoietic cells originating from a non-human mammal M2, having a genetically determined hematopoietic deficiency.

The chimeric non-human mammal M4 of the invention is established by a method comprising destroying the immune system of a non-human mammal M1 and transplanting said treated mammal M1 with tissue or cells other than normal hematopoietic cells originating from a mammal M3 of a species other than that of mammal M1, and additionally tranplanting said treated mammal with hematopoietic cells originating from a non-human mammal, M2, of a species other than that of M3, having a genetically-determined hematopoietic deficiency. In an alternative method, the mammal M1 is first transplanted with bone marrow of mammal M2 and then with tissue or cells of mammal M3.

In a preferred embodiment of the present invention, the non-human receipient mammal, M1, is a mouse, the non-human mammal donor, M2, is a SCID mouse, and the mammal donor M3 is human.

In another embodiment, the present invention provides a method for chemotherapy sensitivity testing of a drug against a human solid tumor or leukemia, which comprises treating a chimeric mammal M4 of the present invention to which cells of said solid tumor or leukemia have been transplanted, with the drug, and monitoring the efficacy of the treatment. In this way, anticancer drugs can be evaluated in vivo in these models. In one embodiment, the mammal will be transplanted with cells originating from a solid tumor or leukemia of patient and the anticancer activity of a drug or of a combination of drugs in the treatment of the same patient will be evaluated in the transplanted chimeric mammal.

The non-human mammal, M1, of the present invention may be any mammal, such as a horse, sheep, or rodent, such as, for example, rabbit, hamster, guinea pig, rat, mouse, etc. It may be any strain of mice. Preferably M1 is a normal mouse, such as BALB/c mouse, or a transgenic mouse carrying human genes, such as human cytokine genes or human HLA determinants. M1 may also be an immune deficient mouse, such as nude mouse, CBA/N and Bg/Nu/Xid mouse. Thus, with this approach, engraftment of human tissues need not be confined to a few immunedeficient strains of mice, such as SCID or nude mice, but may be extended to every normal strain of mice. In addition, immunedeficient strains of mice can be transformed into new chimeras with a broader immune deficiency, for example by irradiaton of Bg/Nu/Xid or nude mice, followed by transplantation with SCID bone marrow and human tissue or cells according to the invention.

The mammal donor M2 having a genetically-determined hematopoietic deficiency may be of the M1 species or of a different mammalian species. Preferably, M1 and M2 are of the same species.

According to the invention, any non-human mammal with a genetically-determined hematopoietic deficiency may be used as the donor, M2. Useful mouse strains with genetically-determined hematopoietic deficiencies include the immune-deficient Bg/Nu/Xid mouse, and the erythroid-deficient W/W$^v$ mouse. Other mouse and mammalian strains will certainly be identified or created by researchers, and will be suitable for use according to the present invention, either as M1 or M2.

Depending on the nature of the genetically-determined hematopoietic deficiency of the mammal M2, the transplanted pluripotent stem cells originating from mammal M2 will reconstitute the hematopoietic cell lineages of the mammal M1, except for the lineages which cannot be produced due to the amount of the hematopoietic deficiency of M2.

The mammal M3 is of a species other than M1, and is preferably human.

Thus, in a preferred embodiment of the present invention, a lethally irradiated mouse is transplanted with human solid tumor or leukemia cells and SCID mouse bone marrow cells, leading to production of a mouse having human malignant cells from a cancer, such as colon, breast, ovary, pancreas, lung, stomach, kidney or prostate, or melanoma, neuroblastoma or glioblastoma, or cancer cells from a leukemia, such as acute or chronic myeloid, lymphatic or monocytic leukemia.

A method by which the stable non-human chimeric mammal, M4, may be produced according to the present invention comprises the following steps:

(i) treating a non-human mammal M1 so as to essentially destroy its immune system;

(ii) transplanting the treated mammal M1 with tissue or cells other than normal hematopoietic cells originating from a mammal M3 of a species other than that of mammal M1, the mammal M3 being preferably human: and

(iii) additionally transplanting bone marrow cells or any other suitable source of pluripotent stem cells from a non-human mammal donor M2, preferably of the same species as M1, and having a genetically-determined hematopoietic deficiency,

thereby obtaining the chimeric non-human mammal M4 bearing hematopoietic cell lineages derived from mammal donor M2 and M3-derived (e.g. human) tissue or cells.

In an alternative method for production of the chimeric non-human mammal M4, the steps (ii) and (iii) above are interchanged so that the treated mammal M1 of step (i) is first transplanted with bone marrow of an immunedeficient mammal M2. The chimeric mammal thus obtained is long-term stable and can be kept ready for further transplantation with tissue or cells from mammal M3 for several weeks.

Although the mammal M3 contributing the tissue or cells to chimeric mammal M4 is preferably human, other mammals such as bovine or equine species may be used. Thus, for example, a chimeric mouse having long-term stable bovine malignant cells may be produced according to the present invention.

The treatment in step (i) for suppression or destruction of the immune system of mammal M1 may include irradiation (e.g., gamma irradiation), chemotherapy, or a combination of both, or cytoreduction with monoclonal antibodies directed against hematopoietic cells, provided that the treatment allows successful repopulation with pluripotent stem cells from mammal M2.

The source of human malignant cells may be primary tumors or metastatic tissue dissected by surgery, or leukemic cells. The human tissues, e.g., thymus, liver, spleen, pancreas, lymph nodes, etc., are obtained from healthy or unhealthy subjects and grafted into mammal M1 as small pieces.

The chimeric mammal M4 of the present invention, having malignant tumor cells, may be used as an experimental model for the study of a variety of human solid tumors and leukemias, providing a tool for chemotherapy sensitivity testing of drugs.

The chimeric mammal M4 of the invention having human tissue may be used as an experimental model for the study of a variety of human specific diseases, such as virus infections and autoimmune diseases, being useful tool for screening, sensitivity testing and monitoring of drugs for the diseases. Thus, grafting of human liver tissue will establish a model for study of hepatitis B and C; of thymus, spleen or lymph nodes tissue will enable the study of AIDS, and grafting of pancreas tissue will establish a model for diabetes mellitus.

According to the invention, it is possible to test compounds or biomolecules for efficacy against human tumors established from fresh specimens removed during surgery. It is anticipated that tumors from the vast majority of human cancer patients will grow in a human:mouse chimera of the invention, and this will allow the testing of a large number of new drugs against tumors, under conditions approaching in vivo testing in humans. In addition to testing new drugs for anti-tumor activity, it will be possible to test established chemotherapeutic agents and their combinations for anti-tumor activity against tumor of an individual, to identify the most effective agents to treat such a patient.

In a further embodiment, IL-2 activated peripheral blood mononuclear cells also referred to as lymphokine activated killer cells (LAK) (Rosenberg, S.A. et al. (1985) J. Exper. Med. **161**: 1169-1188, and (1987) Engl. J. Red. **316**: 899-941; Lotze M.T. et al. (1980) J. Immunol.**125**: 2972-2978; Lotze, M.T. et al. (1981) Cancer Res. **41**: 4420-4425; Grimm, E.A. et al (1983) J. Exper. Red. **158**: 1365-1371), or tumor infiltrating lymphocytes (TIL) (Rosenberg, S.A. et al. (1988) N. Engl. J. Red. **319**: 1676-1680) are transplanted into a mammal, e.g. a mouse, M4 bearing a human tumor, so as to test the efficacy of the LAK or TIL cells in immunotherapy, optionally together with a cytokine, such as IL-2.

The invention will now be illustrated by the following examples, herein presented in a non-limiting manner.

## EXAMPLES

### Example 1.

Engraftment of human melanoma cells into BALB/c mice

Eight to twelve week-old female BALB/c mice (obtained from Olac Farms, Bicester, England) were exposed to a single dose of 10 Gy TBI from a Gamma beam 150-A $^{60}$Co source with a focal skin distance (F.S.D.) of 75 cm, at a 0.7-0.9 Gy/min dose rate. One day later, 1-5 x $10^6$ melanoma cells prepared from dissected primary tumor as described by Naito, S. et al. (Invasion Metastasis (1987) 7: 16-29) were transplanted subcutaneously into each mouse. Twenty four hours later, T cell-depleted bone marrow cells (2 x $10^6$) from 8 to 12 week-old male SCID mice (obtained from the Weizmann Institute Animal Breeding Center), prepared according to Reisner et al. (Reisner, Y., et al. (1978) Proc. Natl. Acad. Sci. USA **75**, 2933), with minor modifications (Schwartz, E., et al. (1987) J. Immunol. **138**, 460), were transplanted into the treated mice by intravenous infusion on the second day post TBI. Tumor growth was followed daily by visualization of pigmented cells. Within an observation period of three months, 10 out of 12 engrafted evaluable mice, that survived the initial 30 days post-bone marrow transplantation, have developed primary tumors. Spontaneous metastasis was observed in 2 out of the 10 mice.

### Example 2. Engraftment of human colon, breast or leukemia cells into BALB/c mice

Human tumor cells from established cell lines of colon, breast, or different types of leukemia, were accepted for more than 30 days when grafted (1 x $10^6$ cells per mouse) subcutaneously into lethally irradiated BALB/c mice before or after transplantation of T cell-depleted SCID bone marrow cells (1 x $10^6$), prepared as in Example 1. Within an observation period of 3 months, several mice developed primary tumors : colon (13/17), breast (6/12) and leukemia (K562, 4/4).

### Example 3. Transplantation of Organs

Small pieces (approximately 1 cm$^2$) of human tissue : thymus, liver, spleen, pancreas and lymph node were grafted under the kidney capsules of BALB/c mice, as described by Nakamura et al., cited above, two weeks after TBI irradiation of the BALB/c mice (Day 0) followed by transplantation of SCID bone marrow (1 x $10^6$ cells) (Day 1). Takes of grafted human tissues are evaluated by histology and/or by immunohistochemistry. Immunohistological tests of thymus implants suggest that, within an observation period of one month after transplant, the stroma structure of human thymus implants was accepted and maintained under the kidney capsules of the BALB/c mice.

## Claims

1. A non-human chimeric mammal M4 having tissue or cells other than normal hematopoietic cells originating from a mammal M3, said mammal M4 being originated from a mammal M1 whose hematopoietic cells have been substantially suppressed or destroyed and replaced by hematopoietic cells originating from a mammal M2, of a species other than that of M3, having a genetically-determined hematopoietic deficiency, and to which mammal M1 tissue or cells of mammal M3 have been transplanted.

2. The non-human chimeric mammal M4 according to claim 1 wherein said mammal M3 is a human.

3. The non-human chimeric mammal M4 according to claim 1 or 2 having human malignant cells derived from leukemia or solid tumor cells.

4. The non-human chimeric mammal according to claim 1 or 2 having tissue of human organs.

5. The non-human chimeric mammal M4 according to any of claims 1 to 4 wherein said mammal M2 and said mammal M1 are of the same mammalian species.

6. The non-human chimeric mammal M4 according to claim 5 wherein both mammals M2 and M1 are mice.

7. The non-human chimeric mammal according to claim 6 wherein said mammal M2 is a mouse with severe combined immunodeficiency (SCID).

8. The non-human chimeric mouse according to claim 6 or 7 wherein said mammal M1 is a normal mouse, including BALB/c, transgenic or an immunodeficient mouse other than SCID mouse.

9. A method for the production of a non-human chimeric mammal M4 according to any of claims 1 to 8, which comprises:
    (a) treating a non-human mammal M1 so as to essentially destroy its immune system;
    (b) transplanting said treated mammal M1 with tissue or cells other than normal hematopoietic cells originating from a mammal M3 of a species other than that of mammal M1; and
    (c) additionally transplanting said treated mammal of step (b) with hematopoietic cells originating from a mammal M2, of a species other than that of M3, having a genetically-determined hematopoietic deficiency,
    thereby obtaining said chimeric non-human mammal M4 having hematopoietic cells derived from mammal donor M2 and further having tissue or cells other than normal hematopoietic cells from mammal donor M3.

10. A method for the production of a non-human chimeric mammal M4 according to any of claims 1 to 8, which comprises:
    (a) treating a non-human mammal M1 so as to essentially destroy its immune system;
    (b) transplanting said treated mammal of step (a) with hematopoietic cells originating from a mammal M2, of a species other than that of M3, having a genetically-determined hematopoietic deficiency; and
    (c) additionally transplanting said treated mammal M1 of step (b) with tissue or cells other than normal hematopoietic cells originating from a mammal M3 of a species other than that of mammal M1;
    thereby obtaining said chimeric non-human mammal M4 having hematopoietic cells derived from mammal donor M2 and further having tissue or cells other than normal hematopoietic cells from mammal donor M3.

11. The method according to claim 9 or 10 wherein the treatment of step (a) comprises chemotherapy, lethal irradiation or a combination of chemotherapy and lethal irradiation.

12. The method according to any one of claims 9 to 11 wherein said mammal M3 is a human and said cells transplanted in step (b) or (c), respectively, are human malignant cells selected from the group consisting of solid tumor cells and leukemia cells.

13. A method according to any one of claims 9 to 11 wherein said mammal M1 is a mouse ,said mammal M2 is a mouse, said mammal M3 is a human, and said cells transplanted in step (b) or (c), respectively, are human malignant cells from colon, breast, ovary, pancreas, lung, stomach, kidney or prostate cancer, or melanoma, neuroblastoma or glioblastoma, or from acute or chronic myeloid, lymphatic or monocytic leukemia.

14. A method for the production of a chimeric mammal according to claim 7 having engrafted human malignant cells and hematopoietic cells derived from a SCID mouse, which comprises:
    (a) lethally irradiating a recipient mouse such as to essentially destroy its immune system;
    (b) transplanting said irradiated mouse with human malignant cells; and
    (c) additionally transplanting said transplanted mouse of step (b) with T cell-depleted SCID mouse bone marrow cells,
    thereby obtaining a chimeric mouse having human malignant cells.

15. A method for chemotherapy sensitivity testing of a drug against a human solid tumor or leukemia which comprises treating a chimeric mammal according to any of claims 1 to 8 having said human solid tumor or leukemia cells with the drug, and monitoring the efficacy of said treatment.

16. The method according to claim 15 for testing the chemotherapy activity of a drug against an individual tumor, or leukemia, wherein the chimeric mammal has been transplanted with cells originating from the tumor or leukemia of said individual.

European Patent
Office

**PARTIAL EUROPEAN SEARCH REPORT**
which under Rule 45 of the European Patent Convention
shall be considered, for the purposes of subsequent
proceedings, as the European search report

Application Number

EP  92 10 9402

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X Y,D | EP-A-0 438 053 (YEDA RESEARCH AND DEVELOPMENT CO., LTD) * Page 6, lines 42-51; whole document * --- | 1-14 | C 12 N  15/00 G 01 N  33/574 A 01 K  67/027 |
| X,Y D | SCIENCE, vol. 252, no. 5004, 19th April 1991, pages 427-431, Lancaster, PA, US; I. LUBIN et al.: "Engraftment and development of human T and B cells in mice after bone marrow transplantation" * Page 430; whole document * --- | 1-14 | |
| Y | CANCER RESEARCH, vol. 47, 1st May 1987, pages 2456-2460; S. REDDY et al.: "Human lung tumor growth established in the lung and subcutaneous tissue of mice with severe combined immunodeficiency" * Whole document * ---                     -/- | 1-14 | |

TECHNICAL FIELDS SEARCHED (Int. Cl.5)

C 12 N
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:

Remark: Although claims 15 and 16 are directed to a diagnostic method practised on the animal body the search has been carried out and based on the alleged effects of the compound/composition.

| Place of search | Date of completion of the search | Examiner |
|---|---|---|
| THE HAGUE | 02-09-1992 | CHAMBONNET F.J. |

European Patent
Office

PARTIAL EUROPEAN SEARCH REPORT

Application Number

EP  92 10 9402

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| D,Y | SCIENCE, vol. 242, no. 4885, 23rd December 1989, pages 1706-1709, Lancaster, PA, US; S. KAMEL-REID et al.: "Engraftment of immune-deficient mice with human hematopoietic stem cells" <br> * Page 1708, column 3, lines 21-33 * <br> ----- | 1-14 | |
| | | | TECHNICAL FIELDS SEARCHED (Int. Cl.5) |

EPO FORM 1503 03.82 (P0410)